# EUROPEAN PATENT SPECIFICATION

(11) **EP 1 150 658 B1**
(45) Date of publication and mention of the grant of the patent: **04.08.2004**
(21) Application number: 00907183.8
(22) Date of filing: 03.02.2000
(51) Int. Cl.: A61K 9/127, A61K 9/133

(54) **METHOD FOR CONTROLLING LIPOSOME SIZE**
VERFAHREN ZUR KONTROLLE DER LIPOSOMENGRÖSSE
PROCEDE SERVANT A PREPARER DES LIPOSOMES DE DIMENSIONS SELECTIONNEES

(30) Priority: 08.02.1999 US 119229 P
(43) Date of publication of application: 07.11.2001
(73) Proprietor: Alza Corporation, Mountain View, CA 94039-7210 (US)
(72) Inventor: SLATER, James, L., Palo Alto, CA 94304 (US); ZETTER, Adam, A., Menlo Park, CA 94025 (US); ZHU, George, Z., San Jose, CA 95129 (US)
(74) Representative: Suèr, Steven Johannes
(86) International application number: PCT/US2000/003039
(87) International publication number: WO 2000/045791

(56) References cited:
- WO-A-91/16039
- WO-A-96/39121
- DATABASE WPI Week 199813 Derwent Publications Ltd., London, GB; AN 1998-143653 [13] XP002145573 & RU 2 085 192 C (A. MED. CARDIOLOGICAL RES. CENTRE) 27 July 1997 (1997-07-27)

## Description

### Field of the Invention

The present invention relates to a method for preparing a liposome composition, which provides control over the resulting liposome size. The invention further relates to compositions prepared according to the method.

### Background of the Invention

Liposomes are spherical aqueous particles that are surrounded by at least one fully closed lipid bilayer and typically have a diameter of from about 70 nm to several 1000 nm. Over the last decade, liposomes have become important as vehicles for transporting pharmaceutical and cosmetic agents. Such agents are enclosed within the aqueous compartment or are integrated within the liposome lipid bilayer, depending on the hydrophilicity of the agent.

Methods for preparing liposomes are already known in the art. Multilamellar vesicles, that is liposomes having more than one lipid bilayer, are prepared most simply by depositing the vesicle-forming lipid or lipids from an organic solvent in a thin film on the wall of a flask by rotary evaporation under reduced pressure. An aqueous buffer is added and the lipids are hydrated at a temperature above the crystalline melting temperature of the lipid or above the higher crystalline melting temperature of the highest melting component in the lipid mixture. Addition of the aqueous buffer is accompanied with agitation, *e.g.*, stirring, sonicating, vortexing, to obtain a suspension of liposomes.

After liposome formation, the liposomes are sized to obtain a smaller and/or more uniform distribution of particle size. One common sizing method is extrusion of the liposome suspension through a series of sized filters. Alternatively, the liposomes are sized by sonicating the suspension to break up the liposomes into smaller particles.

Small and large unilamellar vesicles (SUVs and LUVs) are liposomes with one lipid bilayer and can be prepared by a number of techniques. SUVs typically have diameters in the range of 20-50 nm and can be formed by rapid injection of a dilute solution of lipid in ethanol into an aqueous phase, the so-called ethanol injection procedure. Another technique for forming SUVs is by vigorous sonication of multilamellar vesicles. Large unilamellar vesicles can be from several hundred nanometers to several microns in size and are typically formed by a reverse phase evaporation procedure, where the organic solvent from a water-in-oil emulsion of lipid, buffer and organic is removed under reduced pressure.

WO 91/16039 describes a process for the preparation of aqueous liposome suspensions containing active substances, the suspensions being prepared from a solution of a liposome-forming substance, or a mixture of such substances, in a lower alcohol with a maximum of three carbon atoms and an aqueous phase. The active ingredients are dissolved or suspended in the lipid solution and/or the aqueous phase. The process is characterised in that the aqueous phase is incorporated, by mixing, in the alcoholic solution, and the lower alcohol removed by vacuum distillation. If necessary, the liposome suspension freeze-dried.

### Summary of Invention

Accordingly, it is an object of the invention to provide a method for preparing liposomes with a desired diameter.

It is another object of the invention to provide a method for obtaining liposomes having a selected size.

It is a further object of the invention to provide a method for controlling the size of liposomes through selection of the composition of the hydration composition.

According to the present invention there is provided a method for preparing a liposome composition comprising liposomes having a desired size, the method comprising (i) determining an amount of lipid solvent required to achieve the desired liposome size from a profile of liposome size as a function of amount of lipid solvent in a hydration mixture of the lipid solvent and a second solvent with which the lipid solvent is miscible, and (ii) hydrating a vesicle-forming lipid dissolved in said determined amount of lipid solvent with the second solvent to form the hydration mixture, said determined amount of lipid solvent being between 10-50 weight percent.

In one embodiment, the lipid solvent is an alcohol, such as methanol, ethanol or butanol.

The vesicle-forming lipid is, in one embodiment, a charged vesicle-forming lipid, such as the cationic lipid DODAC or the anionic lipid DSPE. In another embodiment, the vesicle-forming lipid is a neutral vesicle lipid.

In yet another embodiment, the hydrating further includes hydrating a vesicle-forming lipid derivatized with a hyprophic polymer chain, such as polyethyleneglycol.

The second solvent in the method of the invention, in one preferred embodiment, is water.

In another embodiment, hydrating includes hydrating a therapeutic agent with the second solvent. Alternatively, hydrating can further include hydrating a therapeutic agent with the lipid solvent.

In a preferred embodiment, the therapeutic agent is a radiosensitizer with one or more acyl chains. One preferred derivatized radiosensitizer is dipalmtoyl 5-iodo-2-dexoyuridine.

In another embodiment, the therapeutic agent is a nucleic acid.

The invention includes a method for obtaining liposomes having a minimum particle size, by mixing a vesicle-forming lipid in a lipid solvent, and adding a second solvent to achieve a lipid solvent amount greater than 10 weight percent and less than about 50 weight percent, where the amount of lipid solvent is selected to obtain liposomes smaller in size than liposomes preparing from a similar formulation except having a lipid solvent amount less than 10 weight percent or greater than 50 weight percent.

In a preferred embodiment, the profile of liposome size as a function of lipid solvent content in the hydration mixture is obtained by measuring the diameter of liposomes in the hydration mixture, and repeating the hydrating and measuring steps at different amounts of lipid solvent to obtain the profile. Based on the profile, the amount of lipid solvent required to achieve a selected liposome particle diameter is selected.

These and other objects and features of the invention will be more fully appreciated when the following detailed description of the invention is read in conjunction with the accompanying drawings.

### Brief Description of the Drawings

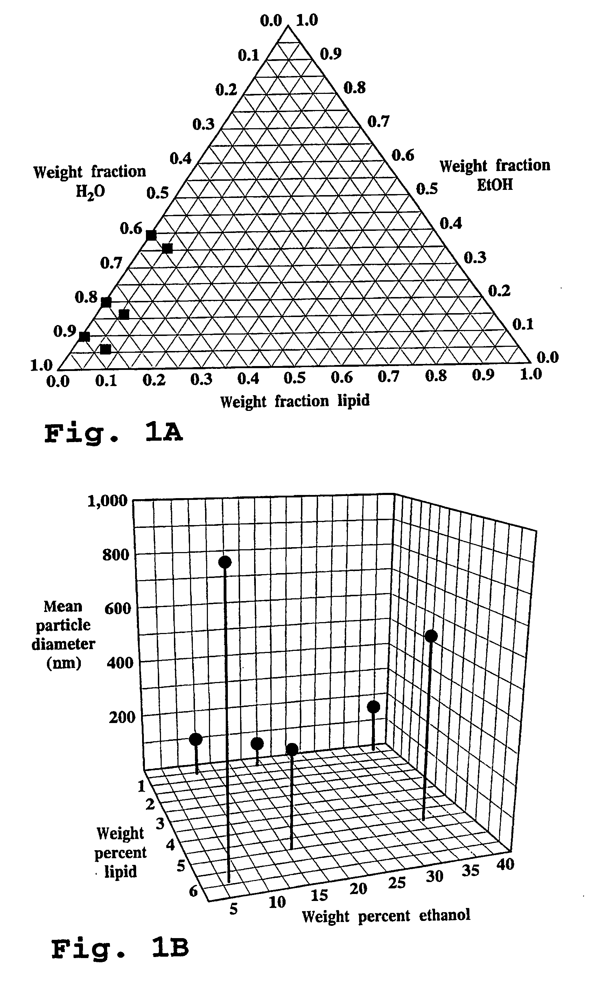

Figs. 1A-1B correspond to liposome suspensions prepared with egg phosphatidylcholine, cholesterol, N,N-dioleoyl-N,N-dimethylammonium chloride (DODAC) and ceramide derivatized with polyethyleneglycol of molecular weight 2000 daltons (C₂₀PEG-ceramide) with an entrapped oligonucleotide, where the composition of each suspension is indicated on the phase diagram of Fig. 1A and the mean particle diameter, in nm, of the liposomes in each suspension are plotted in Fig. 1B as a function of both weight percent lipid and weight percent ethanol;

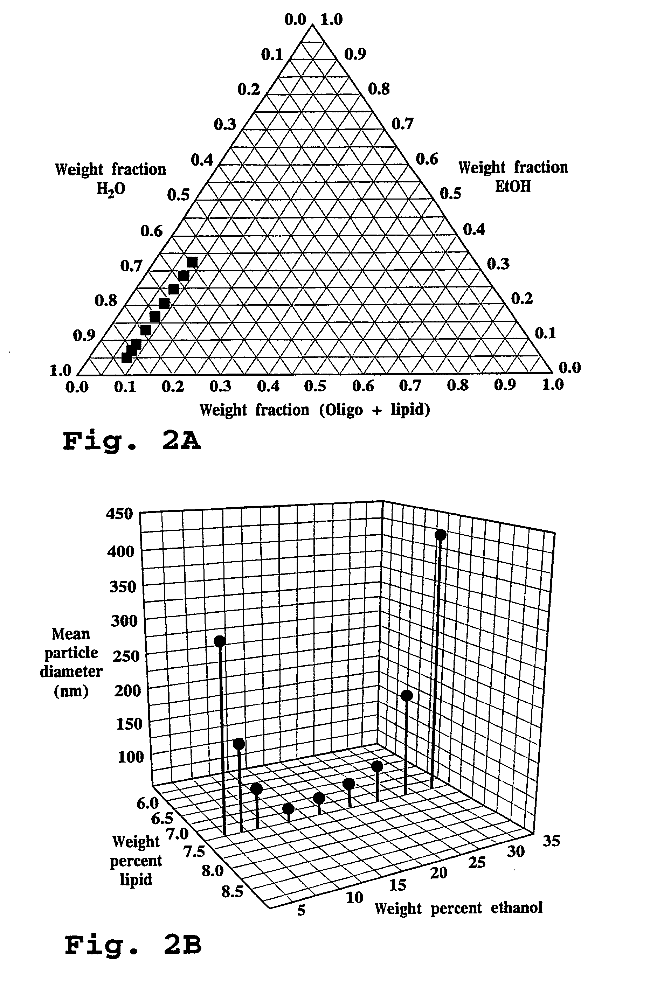

Figs. 2A-2B correspond to liposome suspensions prepared with partially hydrogenated soy phosphatidylcholine, cholesterol, DODAC and a neutral lipid derivatized with mPEG (mPEG-DS) and containing an entrapped oligionucleotide, where the composition of each suspension is indicated on the phase diagram of Fig. 2A and the mean particle diameter, in nm, of the liposomes in each suspension are plotted in Fig. 2B as a function of both weight percent lipid and weight percent ethanol;

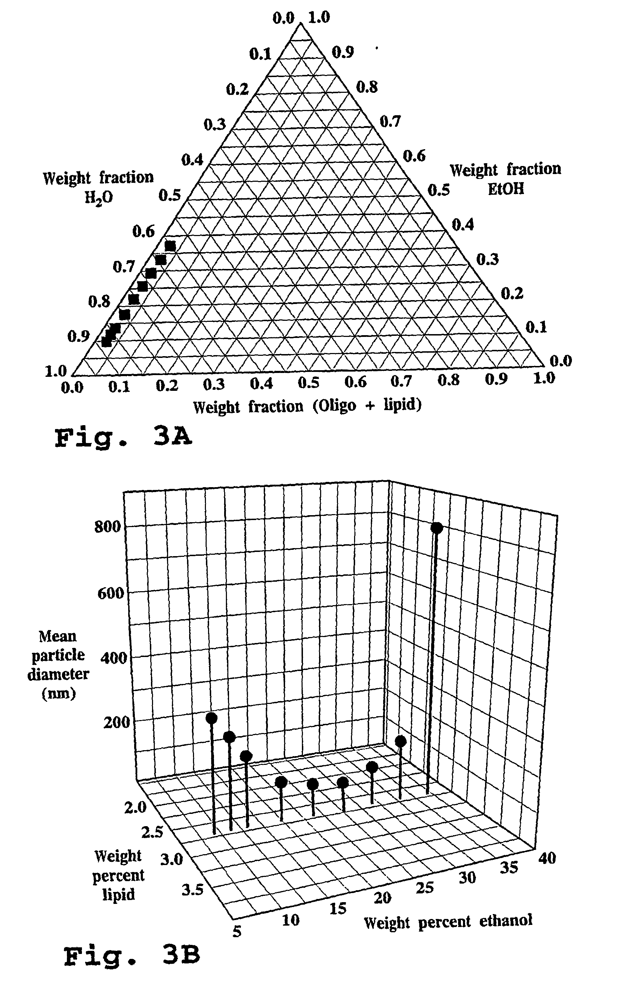

Figs. 3A-3B correspond to liposome suspensions prepared with partially hydrogenated soy phosphatidylcholine, cholesterol, DODAC and an anionic lipid (distearoyl phosphatidyl-ethanolamine, DPSE) derivatized with methoxy-polyethylene glycol (mPEG-DSPE) and having an entrapped oligionucleotide, where the composition of each suspension is indicated on the phase diagram of Fig. 3A and the mean particle diameter, in nm, of the liposomes in each suspension are plotted in Fig. 3B as a function of both weight percent lipid and weight percent ethanol;

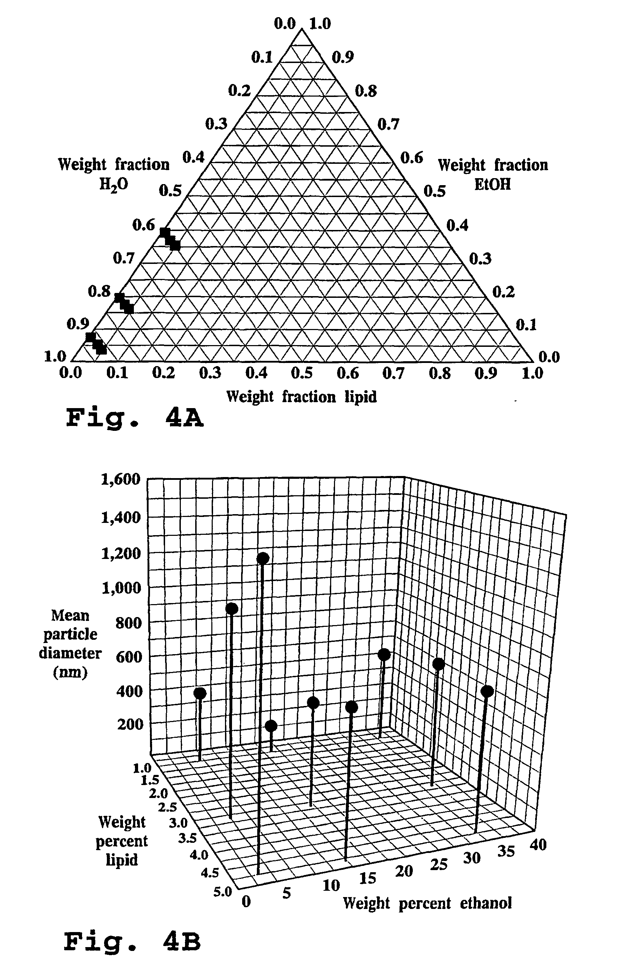

Figs. 4A-4B correspond to liposome suspensions prepared with partially hydrogenated soy phosphatidylcholine, cholesterol and mPEG-DSPE, where the composition of each suspension is indicated on the phase diagram of Fig. 4A and the mean particle diameter, in nm, of the liposomes in each suspension are plotted in Fig. 4B as a function of both weight percent lipid and weight percent ethanol;

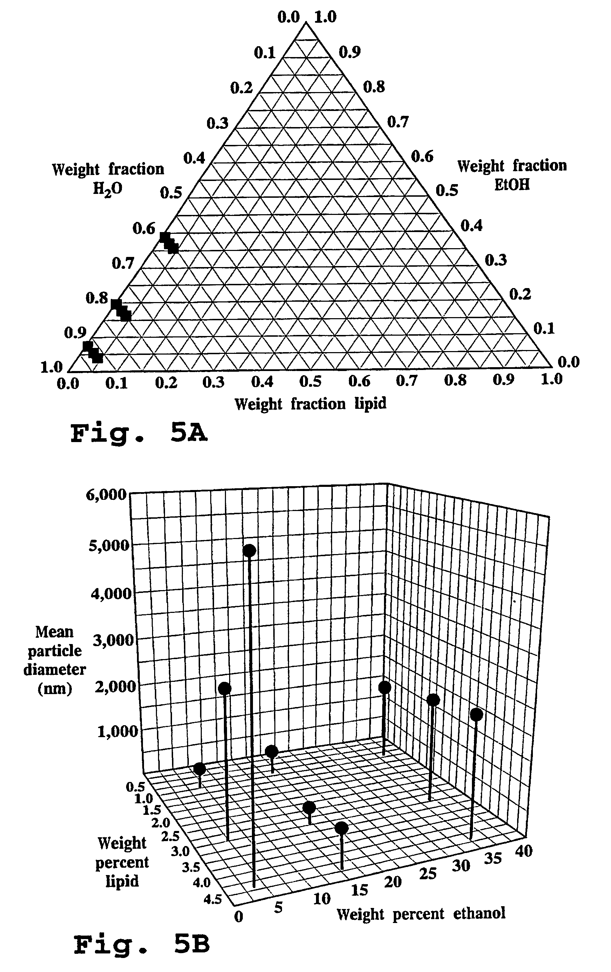

Figs. 5A-5B correspond to liposome suspensions prepared with partially hydrogenated soy phosphatidylcholine and cholesterol, where the composition of each suspension is indicated on the phase diagram of Fig. 5A and the mean particle diameter, in nm, of the liposomes in each suspension are plotted in Fig. 5B as a function of both weight percent lipid and weight percent ethanol;

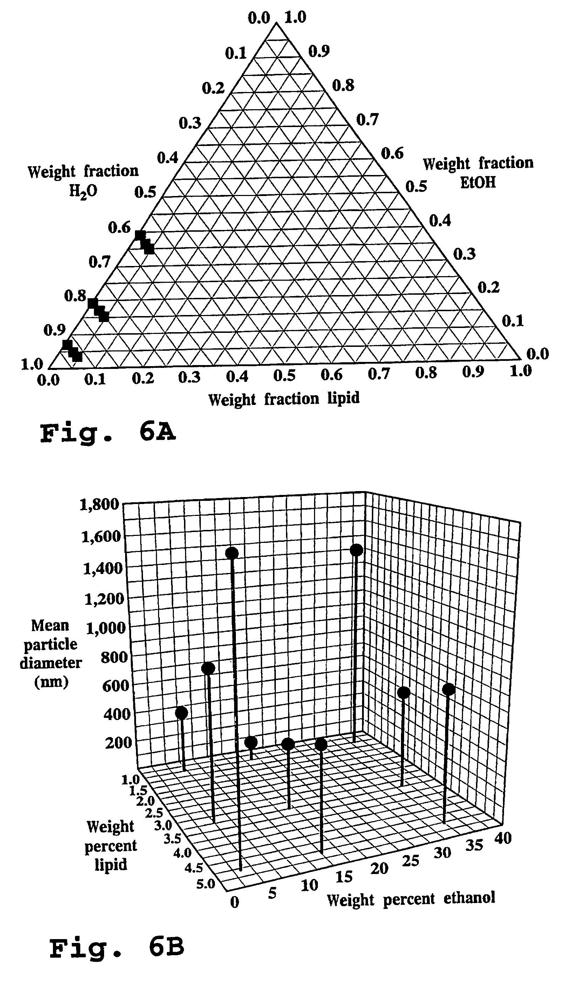

Figs. 6A-6B correspond to liposome suspensions prepared with partially hydrogenated soy phosphatidylcholine, cholesterol and mPEG-DSPE, where the composition of each suspension is indicated on the phase diagram of Fig. 6A and the mean particle diameter, in nm, of the liposomes in each suspension are plotted in Fig. 6B as a function of both weight percent lipid and weight percent ethanol;

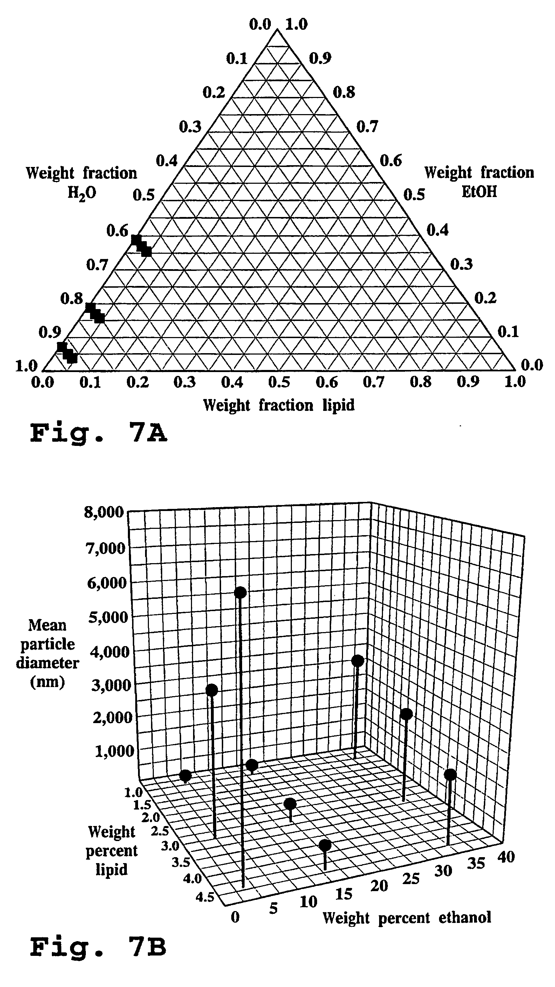

Figs. 7A-7B correspond to liposome suspensions prepared with partially hydrogenated soy phosphatidylcholine and cholesterol, where the composition of each suspension is indicated on the phase diagram of Fig. 7A and the mean particle diameter, in nm, of the liposomes in each suspension are plotted in Fig. 7B as a function of both weight percent lipid and weight percent ethanol;

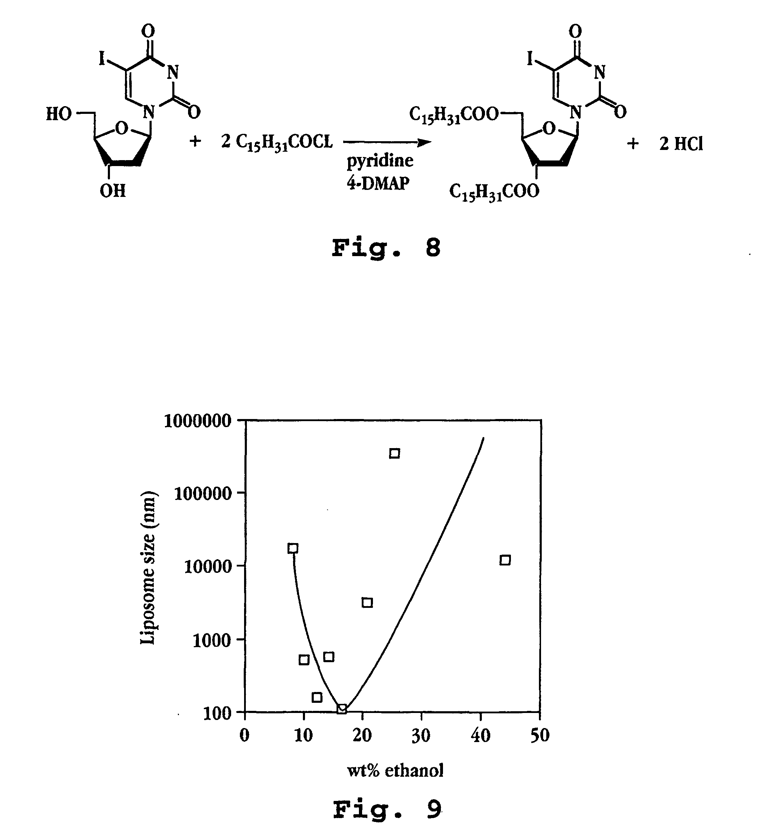

Fig. 8 is a synthetic reaction scheme for synthesis of 3',5'-dipalmitoyl-5-iodo-2'-deoxyuridine;
Fig. 9 is a plot of liposome size, in nm, as a function of weight percent ethanol during hydration of liposome lipids composed of HSPC, mPEG-DSPE and dpIUdR; and

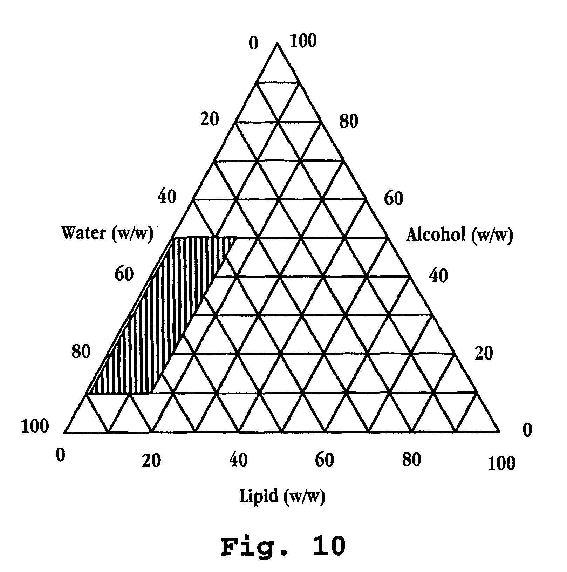

Fig. 10 is a phase diagram for formation of liposomes including dpIUdR in ethanol, water and shows a preferred operating region.

### Detailed Description of the Invention

### I. Definitions and Abbreviations

"Vesicle-forming lipid" refers to any lipid capable of forming part of a stable micelle or liposome composition and typically includes one or two hydrophobic acyl hydrocarbon chains or a steroid group and may contain a chemically reactive group, such as an amine, acid, ester, aldehyde or alcohol, at its polar head group.

"Lipid solvent" refers to any solvent capable of dissolving a vesicle-forming lipid at any temperature.

"Second solvent" refers to a solvent which in at least some proportion is completely or partially miscible with the lipid solvent.

"dpIUdR" refers to 3',5'-dipalmitoyl-5-iodo-2'-deoxyuridine.

"DODAC" refers to the cationic lipid N,N-dioleoyl-N,N-dimethylammonium chloride.

"mPEG-DSPE" refers to distearoyl phosphatidyl-ethanolamine (DPSE) derivatized with methoxy-polyethylene glycol (mPEG). The mPEG can have a molecular weight of between 500-20,000 daltons, preferably between 1,000-5,000 daltons.

"mPEG-DS" refers to a neutral vesicle forming lipid derivatized with mPEG.

### II. Method of the Invention

In one aspect, the invention includes a method of preparing liposomes where the particle size of the liposomes is controlled. In another aspect, the method provides a means for obtaining liposomes having a desired particle size and a means for obtaining a liposomes suspension having a minimum particle size. These aspects will now be described by way of Examples 1-8.

Example 1 describes preparation of a suspension of liposomes, where the lipids forming the liposomes were composed of egg phosphatidylcholine (EPC), cholesterol, DODAC and C₂₀PEG-ceramide in a 50:25:15:10 molar ratio. The lipids were dissolved in ethanol. Aliquots from the mixture of lipids were hydrated with an aqueous solution containing 20 mg/mL of an oligonucleotide 34 bases in length to form six liposome suspensions varying in ethanol concentration. The compositions of the suspensions are indicated on the phase diagram of Fig. 1A, and as seen, are at either approximately 0.6 weight percent lipid or at approximately 6 weight percent lipid. The weight percentages of ethanol at the 0.6% lipid were 9.5, 19.3 and 39. The weight percentages of ethanol at the 6% lipid were 5.9, 15.8 and 35.5. Table 1 in Example 1 summarizes the composition of each liposome suspension.

The mean particle diameter of each suspension was measured by quasi-elastic light scattering just after hydration. The results are reported in Table 1 (see Example 1 below) and are plotted in Fig. 1B against weight percent lipid and weight percent ethanol. As seen in the figure, for each of compositions at the two lipid levels (0.6 wt% and 6 wt%), there is a range of lipid solvent where the liposome diameter is minimized. For the liposomes having approximately 0.6wt% lipids, from about 12-35 wt% ethanol the liposome particle size is less than 100 nm. For the liposomes having approximately 6 wt% lipids, the effect of a minimum particle size at a selected ethanol range is more evident, as the liposome size minimizes between 6-35 weight percent ethanol.

Example 2 provides a further example of the method of the invention, where the particle size of the liposomes is controlled by the composition of the hydration mixture, *e*.*g*., by the amount of lipid solvent and second solvent. In this example liposomes composed of partially hydrogenated soy phosphatidylcholine (PHSPC), cholesterol, DODAC and mPEG-DS in a 50:25:15:10 molar ratio were prepared. The lipids were dissolved in ethanol and hydrated with an aqueous solution containing an oligonucleotide 34 bases in length. Nine liposome suspensions varying the composition of the hydration mixture were prepared, and each composition is shown on the phase diagram in Fig. 2A.

The mean particle diameter of the liposomes in each suspension was measured just after hydration and again after storage overnight at 5 °C. The results are shown in Table 2 below, and the diameters measured just after hydration are plotted in Fig. 2B as a function of weight percent ethanol and weight percent lipid. As seen in the figure, there is a strong relationship between amount of lipid solvent and liposome size. More specifically, there is an interval where the liposome diameter is minimized. This interval, for this particular lipid mixture, falls between 5-30 weight percent ethanol. The smallest liposome size was at 12.9 weight percent ethanol, with the liposome size increasing as the amount of ethanol increased or decreased from 12.9 weight percent.

Accordingly, in one aspect, the invention includes a method for obtaining liposomes having a minimum diameter by conducting a study as set forth in Example 2. That is, a vesicle-forming lipid, or mixture of lipids, are solvated with a lipid solvent and hydrated with a second solvent to form liposomes. The percentage of lipid solvent in the resulting hydration mixture is varied, and the liposome sizes are determined. A profile of liposome size as a function of lipid solvent content is established to determine the composition of the hydration mixture that results in the minimum liposome diameter. It will be appreciated that the profile can be used to determine the minimum liposome diameter at a particular lipid solvent concentration, and also to determine the lipid solvent content in the hydration composition to achieve any desired liposome size.

Further studies were done in support of the invention to examine the effect of the lipid composition on the method. In Example 3, liposomes were formed using a charged, mono-anionic PEG lipid conjugate, mPEG-DSPE. Nine liposome suspensions were prepared and the composition of each is shown on the phase diagram of Fig. 3A. The particle size in each suspension was determined by light scattering, and the values are reported in Table 3, below, and plotted in Fig. 3B. The correlation between liposome size and lipid solvent content is readily apparent in viewing Fig. 3B, where at ethanol percentages between about 15-35 the liposome diameter is minimized.

In Example 1-3, the liposome compositions included a neutral vesicle-forming lipid or an anionic vesicle-forming lipid and a cationic vesicle-forming lipid. For use in the invention, any of the vesicle-forming lipids known to those of skill in the art are suitable, and include those lipids which can form spontaneously into bilayer vesicles in water, as exemplified by the phospholipids, with the hydrophobic moiety in contact with the interior, hydrophobic region of the bilayer membrane, and the head group moiety oriented toward the exterior, polar surface of the membrane.

The vesicle-forming lipids of this type are preferably ones having two hydrocarbon chains, typically acyl chains, and a head group, either polar or nonpolar. There are a variety of synthetic vesicle-forming lipids and naturally-occurring vesicle-forming lipids, including the phospholipids, such as phosphatidylcholine, phosphatidylethanolamine, phosphatidic acid, phosphatidylinositol, and sphingomyelin, where the two hydrocarbon chains are typically between about 14-22 carbon atoms in length, and have varying degrees of unsaturation. The above-described lipids and phospholipids whose acyl chains have varying degrees of saturation can be obtained commercially or prepared according to published methods. Other suitable lipids include glycolipids, cerebrosides and sterols, such as cholesterol.

Cationic lipids are also suitable for use in the liposomes of the invention, where the cationic lipid can be included as a minor component of the lipid composition or as a major or sole component. Such cationic lipids typically have a lipophilic moiety, such as a sterol, an acyl or diacyl chain, and where the lipid has an overall net positive charge. Preferably, the head group of the lipid carries the positive charge. Exemplary cationic lipids include N,N-dioleoyl-N,N-dimethylammonium chloride (DODAC); 1,2-dioleyloxy-3-(trimethylamino) propane (DOTAP); N-[1-(2,3,-ditetradecyloxy)propyl]-N,N-dimethyl-N-hydroxyethylammonium bromide (DMRIE); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3 [N-(N',N'-dimethylaminoethane) carbamoly] cholesterol (DC-Chol); and dimethyldioctadecylammonium (DDAB).

The cationic vesicle-forming lipid may also be a neutral lipid, such as dioleoylphosphatidyl ethanolamine (DOPE) or an amphipathic lipid, such as a phospholipid, derivatized with a cationic lipid, such as polylysine or other polyamine lipids. For example, the neutral lipid (DOPE) can be derivatized with polylysine to form a cationic lipid.

Other lipids which are stably incorporated into lipid bilayers are also contemplated, and include steroids, such as cholesterol.

Also in the Examples 1-3, a vesicle-forming lipid derivatized with a hydrophilic polymer was included in the lipid mixture. As has been described, for example in U.S. Patent No. 5,013,556, including such a derivatized lipid in the liposome composition forms a surface coating of hydrophilic polymer chains around the liposome. The surface coating of hydrophilic polymer chains is effective to increase the *in vivo* blood circulation lifetime of the liposomes when compared to liposomes lacking such a coating. Other hydrophilic polymers conjugated to any of the lipids recited above are suitable, and include polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, polyaspartamide and hydrophilic peptide sequences. The polymers may be employed as homopolymers or as block or random copolymers, as described in U.S. Patent Nos. 5,395,619 and 5,631,018. A preferred hydrophilic polymer chain is polyethyleneglycol (PEG), preferably as a PEG chain having a molecular weight between 500-10,000 daltons, more preferably between 1,000-5,000 daltons. Methoxy or ethoxy-capped analogues of PEG are also preferred hydrophilic polymers, commercially available in a variety of polymer sizes, *e.g.*, 120-20,000 daltons.

Preparation of vesicle-forming lipids derivatized with hydrophilic polymers has been described, for example in U.S. Patent No. 5,395,619. Preparation of liposomes including such derivatized lipids has also been described, where typically, between 1-20 mole percent of such a derivatized lipid is included in the liposome formulation.

The selected vesicle-forming lipid or mixture of lipid is solvated in a "lipid solvent". As used herein, "lipid solvent" refers to an organic solvent in which the lipid components of the liposome are soluble, at any temperature. Examplary lipid solvents include alcohols, such as methanol, ethanol, butanol, etc. and low molecular weight polyols, such as glycerol, propyleneglycol and ethyleneglycol. The lipids are added to the solvent in the desired molar ratio and mixed until dissolved, with heating as necessary. An amount of a second solvent is then added to the lipid solvent mixture to form a hydration mixture. The second solvent, as used herein, refers to a solvent that is miscible with the lipid solvent in some proportion, and preferably is miscible with the lipid solvent in the resulting hydration mixture. The second solvent is added to the lipid solvent mixture in an amount sufficient to bring the weight percentage of the lipid solvent to a selected point between about 10-50 weight percent, more preferably in a lipid solvent weight percent range of 15-45, most preferably in a lipid solvent weight percent range of 20-40, to obtain liposomes having a desired size, as described above.

Examples 4-8 describe further studies using liposome lipid mixtures with no cationic lipid (DODAC) and with no polynucleotide. In Example 4, lipid mixtures of HSPC, cholesterol and mPEG-DSPE in a molar ratio of 50.6:44.3:5.1 were prepared. The mixtures were hydrated with an aqueous solvent to form nine liposome suspensions varying in ethanol content at three lipid concentrations (0.9, 3.1 and 4.7 weight %). The compositions are shown in the phase diagram of Fig. 4A and reported in Table 4 below. The liposome sizes were measured and are plotted in Fig. 4B as a function of lipid and ethanol content. At the three lipid levels, the liposome size is minimized at ethanol concentrations between about 5-35 weight percent ethanol.

Example 5 provides a lipid mixture which does not include a lipid derivatized with a hydrophilic polymer. In Example 5, the lipid mixture of Example 4 except for the mPEG-DSPE (HSPC:cholesterol 53.1:46.9 molar ratio) was used to prepare nine liposome suspensions at lipid weight percentages of 0.9, 2.9 and 4.3. The ethanol content varied between 4-39 weight percent, and the composition of each suspension prepared is indicated on the phase diagram of Fig. 5A. The particle sizes as a function of lipid and ethanol content are shown in Fig. 5B, and as seen, the method of the invention for controlling liposome size through composition of the hydration mixture, and specifically by content of the lipid solvent, is evident even in the absence of the derivatized lipid. As before, between 5-35 weight percent of lipid solvent, the liposome size is correlated to the lipid solvent content.

Example 6 describes preparation of liposome suspensions using a lipid mixture of HSPC, cholesterol and mPEG-DSPE in a molar ratio of 65.4:38.3:5.3. The composition of each suspension is shown in the phase diagram of Fig. 6A and the liposome diameters are plotted in Fig. 6B as a function of lipid and ethanol contents. The liposome size is minimized when the content of ethanol in the hydration mixture is between about 5-35 weight percent. The minimum liposome size at each lipid concentration falls between about 15-20 weight percent ethanol, with the size of the liposomes increasing as the ethanol content increases or decreases from this range.

Example 7 describes preparation of liposome suspensions like those of Example 6, except that the mPEG-DSPE was omitted (HSPC:Chol; 59:41 molar ratio). The phase diagram of Fig. 7A shows the composition of each suspension, and the liposome size is plotted in Fig. 7B, with results similar to those obtained with mPEG-DSPE was present in the lipid mixture. These results and the results of Example 5 show that the control over the liposome size is provided regardless of the type of lipids present.

The method of the invention can be used to prepare liposomes entrapping a variety of therapeutic agents. The therapeutic agent can be mixed with the lipids and the lipid solvent or with the second solvent, depending on the nature of the agent and is solubility in the solvents employed. Agents contemplated are widely varied, and include both therapeutic applications and those for use in diagnostic applications. Therapeutic agents include natural and synthetic compounds having the following therapeutic activities: anti-angiogenesis, anti-aging, anti-arthritic, anti-arrhythmic, anti-bacterial, anticholinergic, anticoagulant, antidiuretic, antidote, antiepileptic, antifungal, antiinflammatory, antimetabolic, antimigraine, antineoplastic, antiparasitic, antipyretic, antiseizure, antisera, antispasmodic, analgesic, anesthetic, beta-blocking, biological response modifying, bone metabolism regulating, cardiovascular, diuretic, enzymatic, fertility enhancing, growth-promoting, hemostatic, hormonal, hormonal suppressing, hypercalcemic alleviating, hypocalcemic alleviating, hypoglycemic alleviating, hyperglycemic alleviating, immunosuppressive, immunoenhancing, muscle relaxing, neurotransmitting, parasympathomimetic, sympathominetric plasma extending, plasma expanding, psychotropic, thrombolytic and vasodilating.

In a preferred embodiment, the entrapped agent is a cytotoxic drug, that is, a drug having a deleterious or toxic effect on cells. Exemplary cytotoxic agents include the anthracycline antibiotics such as doxorubicin, daunorubicin, epirubicin and idarubicin, and analogs of these, such as epirubidin and mitoxantrone; platinum compounds, such as cisplatin and carboplatin.

The entrapped agent can also be a nucleic acid, including ribonucleic acid or synthetic analogues, analogues derivatized to peptides or containing one or more synthetically modified bases or base analogues, or containing a ligand incorporated as part of the sequence including plasmid DNA, synthetic oligonucleotides or other oligo analogues.

It will further be appreciated that the liposomes can include a targeting ligand covalently attached to the free distal end of the hydrophilic polymer chain, which is attached at its proximal end to a vesicle-forming lipid. Alternatively, for composition lacking a lipid derivatized with a hydrophilic polymer, targeting ligands can be attached directly to the liposome outer surface by attaching to a surface lipid component. There are a wide variety of techniques for attaching a selected hydrophilic polymer to a selected lipid and activating the free, unattached end of the polymer for reaction with a selected ligand, and in particular, the hydrophilic polymer polyethyleneglycol (PEG) has been widely studied (Allen, T.M., *et al*., *Biochemicia et Biophysica Acta* 1237:99-108 (1995); Zalipsky, S., *Bioconjugate Chem*., 4(4):296-299 (1993); Zalipsky, S., *et al*., *FEBS Lett.* 353:71-74 (1994); Zalipsky, S., *et al*., *Bioconjugate Chemistry,* 705-708 (1995); Zalipsky, S., in STEALTH LIPOSOMES (D. Lasic and F. Martin, Eds.) Chapter 9, CRC Press, Boca Raton, FL (1995)).

Generally, the PEG chains are functionalized to contain reactive groups suitable for coupling with, for example, sulfhydryls, amino groups, and aldehydes or ketones (typically derived from mild oxidation of carbohydrate portions of an antibody) present in a wide variety of ligands, such as those set forth in PCT Application No. 98/16202 and which are incorporated by reference herein. Examples of such PEG-terminal reactive groups include maleimide (for reaction with sulfhydryl groups), N-hydroxysuccinimide (NHS) or NHS-carbonate ester (for reaction with primary amines), hydrazide or hydrazine (for reaction with aldehydes or ketones), iodoacetyl (preferentially reactive with sulfhydryl groups) and dithiopyridine (thiol-reactive). Synthetic reaction schemes for activating PEG with such groups are set forth in U.S. Patent Nos. 5,631,018, 5,527,528, 5,395,619, and the relevant sections describing synthetic reaction procedures are expressly incorporated herein by reference.

Example 8 describes preparation of a liposome composition containing the radiosensitizer dpIUdR. 5-iodo-2'deoxyuridine, referred to herein as IUdR, was derivatized with a 16-carbon fatty acid, palmitic acid, at two positions on the ribose sugar of IUdR, as shown in Fig. 8. In the reaction scheme shown in the figure and detailed in Example 8A, IUdR was reacted with a small excess of palmitoyl chloride and 4-dimethylpyridine catalyst in pyridine or in pyridine/chloroform as the solvent to yield 3',5'-dipalmitoyl-5-iodo-2'-deoxyuridine, referred to herein as dpIUdR.

As described in Example 8B, the lipids HSPC, mPEG-DSPE and dpIUdR were dissolved in ethanol in an 89:5:6 molar ratio. This lipid stock solution was used to prepare liposome suspensions by hydrating an amount of the lipid solution with a second solvent, an aqueous buffer. Liposome suspensions were prepared in triplicate at final ethanol weight percentages of 8.1, 10.1, 12.2, 14.3, 16.5, 20.8, 25.3, and 44.1. The average size of the liposomes in each sample was measured by quasi-elastic light scattering, and the results are shown in Table 8.

**Table 8**

| **Sample No.** | **Liposome Size in nm at Weight Percent Ethanol** | | | | | | | |
|---|---|---|---|---|---|---|---|---|
| | **8.1** | **10.1** | **12.2** | **14.3** | **16.5** | **20.8** | **25.3** | **44.1** |
| 1 | 51200 | 1410 | 226 | 197 | 84 | 53700 | 1000000 | 12500 |
| 2 | 71 | 46 | 78 | 119 | 149 | 27300 | 11000 | 10500 |
| 3 | 405 | 78 | 157 | 1360 | 84 | 11400 | 21400 | 12500 |
| **AVERAGE** | 17225 | 511 | 154 | 559 | 106 | 30800 | 344133 | 11833 |

In viewing the last row of Table 8, it is apparent that there is a minimum in the liposome particle size at 16.5 weight percent ethanol. This data is shown graphically in Fig. 9 and the trough in liposome size beginning at about 10 weight percent and ending at about 25 weight percent is apparent. A profile such as the one in Fig. 9 of lipid size as a function of ethanol content can be employed to determine the ethanol amount needed to obtain a particular liposome size. For example, the minimum particle size of 106 nm is obtained by hydrating the lipid-ethanol mixture to 16.5 weight percent ethanol. A larger particle size is obtained by hydrating to achieve more or less ethanol, according to the profile. Establishing such a profile, the target amount of lipid solvent to achieve a minimum particle size or a selected particle size is determined for any mixture of lipids and solvents, as will be further illustrated below.

Fig. 10 is a phase diagram showing the operating region for formation of the liposomes in accordance with the invention. In the diagram, the shaded region corresponds to formation of liposomes where the lipid solvent amount is between about 10-50 weight percent and the weight percent of the lipids is between 0.1-15. Depending on the lipid mixture and the lipid solvent, the point at which a minimum in liposome particle size occurs within the operating region can be determined. The liposome suspensions in the operating region are visually clear and contain submicron size liposomes. It will be appreciated that the operating region will vary slightly according to the lipid, solvent and drug components. One of skill in the art can readily conduct a study like that set forth in Example 8B and in Table 8 to determine the amount of lipid solvent that yields a minimum in the liposome size.

Liposomes formed by the above-described method can be, depending on the lipid solvent amount in the hydration mixture, at a minimum particle size. Thus, in some cases, it may not be necessary to further size the liposomes via extrusion or other technique. In some cases, it may be desirable to further process the liposomes, for example by extrusion. The method of preparation is particularly useful for incorporation of lipid-derivatized drugs into liposomes at a high drug-to-lipid ratio, where obtaining a pharmaceutically useful particle size of between 90-150 nm is difficult due to an inability to extrude the mixture, as discussed above. Formation of liposomes according to the invention overcomes this limitation, since, the liposomes are at or near to the desired particle size upon hydration with the second solvent, and any further size processing is minimized. Thus, a higher lipid-derivatized drug load can be employed while still being able to achieve the desired liposome size.

This feature of the invention is illustrated by the study described in Example 8C. Liposomes were prepared using the lipids HSPC, mPEG-DSPE and dpIUdR, where the molar amounts of the formulations were 89/5/6, 85/5/10, 80/5/15, 70/5/25 and 55/5/40. The lipids, including the dpIUdR, were dissolved in ethanol and then hydrated with sufficient water to bring the ethanol concentration in each mixture to 16.5 weight percent (20 volume percent). Each liposome suspension was then extruded as described in the Example and after extrusion, the average particle size of the liposomes in each suspension was measured. The results are shown in Table 9.

**Table 9**

| **Mole percent dpIUdR** | **Conc. dpIUdR Drug/lipid ratio (mg/ml)** | | **Liposome size (nm)** |
|---|---|---|---|
| 6 | 1.25 | 1.67 | 99 |
| 10 | 2.32 | 3.17 | 95 |
| 15 | 4.09 | 4.65 | 103 |
| 25 | 6.90 | 8.91 | 142 |
| 40 | - | - | -* |

| | | | |
|---|---|---|---|
| *composition could not be extruded | | | |

Liposomes containing 6, 10 and 15 mole percent dpIUdR were readily sized to about 100 nm when prepared according to the method of the invention by hydrating the lipid mixture to an amount of lipid solvent that gives a minimum particle size. Importantly, the liposomes are formed at the minimum particle size with a drug-to-lipid ratio of between about 1.5 and 5, more preferably between 2-4. In a preferred embodiment of the invention, liposomes having the desired particle size are prepared using the method to a drug-to-lipid ratio of greater than about 4, as achieved with the liposome composition having 15 mole percent dpIUdR.

From the foregoing it can be appreciated how various objects and features of the invention are met. Preparation of liposome according to the method provides a means of controlling the size of the particles in the suspension. More specifically, there is a relationship between liposome size and composition of the hydration mixture, *i. e.*, amount of lipid solvent in the hydration mixture, at greater than about 10 weight percent and less than 50 weight percent lipid solvent. In this region, there is a minimum in the liposome

### III. Examples

The following examples illustrate the method of controlling liposome particle size via composition of the hydration mixture. The examples are in no way intended to be limiting to the scope and spirit of the invention.
Materials: The following materials were obtained from the indicated source: egg phosphatidylcholine (Avanti Polar Lipids, Alabaster, AL); partially hydrogenated soy phosphatidylcholine (Vernon Walden Inc., Green Village, NJ); cholesterol (Solvay Pharmaceuticals, The Netherlands); N,N-dioleoyl-N,N-dimethylammonium chloride (DODAC) (Northern Lipids, Canada); PEG (MW 2000)-ceramide conjugate (C₂₀PEG ceramide) (Northern Lipids, Canada); histidine (Seltzer Chemicals, Carlsbad, CA); ethanol (Quantum Chemical Co. Tuscola, IL). mPEG-DSPE was prepared as described in the literature (for example, Zalipsky, *S., et al*, *Bioconjugate Chem*., 4:296-299 (1993)).

### Methods

QUELS: quasi-elastic light scattering was performed using a Coulter model N4MD (Coulter Corporation, Miami, FL) for Example 1; and a Brookhaven Instruments Model B1-200SM (Brookhaven Instruments Corporation, Holtsville, NY) for Examples 2-7.

### Example 1

1975 mg of egg phosphatidylcholine, 483.8 mg of cholesterol, 436.9 mg of DODAC and 1337.5 mg of C₂₀PEG ceramide were mixed with 5 ml of ethanol and heated to about 65-70 °C until dissolved. The composition of this lipid stock solution was 51.7 weight percent lipid and 48.3 weight percent ethanol. The total lipid concentration was 496 mmoles/mL.

An oligonucletide having approximately 34 bases was dissolved in 10 mmolar histidine, 0.9% NaCl (pH 6.5) to a concentration of 20 mg/mL.

Liposome suspensions were formed by mixing aliquots of the lipid solution with aliquots of the oligonucleotide stock solution. Both stock solutions were diluted as needed with their respective solvents just prior to mixing, in order to vary the ethanol concentration of the mixture while maintaining a fixed oligonucleotide and lipid concentration. Six liposome suspensions were formed from the stock solutions, and the composition of each mixture is indicated on the phase diagram of Fig. 1A.

After mixing the mean particle diameters of the liposomes in the suspension were determined by Quasi-elastic light scattering. The results are shown in Table 1.

**Table 1**

| **Lipid after mixing wt%** | **Ethanol after mixing wt%** | **Mean Particle Diameter(nm) 90°/30° scattering** |
|---|---|---|
| 4.6 | 35.5 | 628/681 |
| 5.0 | 15.8 | 322/357 |
| 5.9 | 5.9 | 932/767 |
| | | |
| 0.46 | 39.0 | 183/381 |
| 0.50 | 19.3 | 87/161 |
| 0.59 | 9.5 | 136/243 |

Fig. 1B is a plot showing the mean particle diameter in nm as a function of both weight percent lipid and weight percent ethanol.

### Example 2

1183 mg of partially hydrogenated soy phosphatidylcholine, 290 mg of cholesterol, 262 mg of DODAC and 795 mg of mPEG-DS were mixed with 0.47 mL of ethanol and heated until dissolved. The composition of this lipid stock solution was 87.2 weight percent lipid and 12.8 weight percent ethanol.

A stock solution containing 20 mg/mL of a 34 base oligonucleotide was prepared in 10 mmolar histidine, 0.9% NaCl, pH 6.5 aqueous solution.

Aliquots of the lipid stock solution, diluted as needed with ethanol, were hydrated with aliquots of the aqueous oligonucleotide solution, diluted as needed with the aqueous phase, were taken to prepare suspensions of liposomes at the compositions indicated in Fig. 2A.

Each liposome suspension was characterized by measuring the mean particle diameter using QELS just after hydration and after overnight incubation at 5 °C. The results are shown in Table 2.

**Table 2**

| **Sample** | **Post hydration weight percent EtOH** | **Post hydration weight percent Lipid** | **Post hydration weight percent Oligonucleotide** | **Mean diameter (nm) after mixing 90 degree/30 degree scattering** | **Mean diameter (nm) after overnight incubation at 5 degrees after mixing 90 degree/30 degree scattering** |
|---|---|---|---|---|---|
| 1 | 32.6 | 7.2 | 1 | 425/534 | 1037/660 |
| 2 | 28.7 | 7.2 | 1 | 200/258 | 275/421 |
| 3 | 24.7 | 7.2 | 1 | 101/173 | 154/202 |
| 4 | 20.8 | 7.2 | 1 | 84/167 | 100/165 |
| 5 | 16.9 | 7.2 | 1 | 72/145 | 72/123 |
| 6 | 12.9 | 7.2 | 1 | 66/129 | 67/127 |
| 7 | 9.0 | 7.2 | 1 | 104/317 | 105/360 |
| 8 | 7.0 | 7.2 | 1 | 172/406 | 177/430 |
| 9 | 5.0 | 7.2 | 1 | 310/517 | 323/556 |

Fig. 2B is a plot showing the mean particle diameter in nm as a function of both weight percent lipid and weight percent ethanol.

### Example 3

394 mg of partially hydrogenated soy phosphatidylcholine, 96.6 mg of cholesterol, 87.4 mg of DODAC and 275 mg of mPEG-DSPE were mixed with 2.147 mL of ethanol and heated until dissolved. The composition of this lipid stock solution was 33.5 weight percent lipid and 66.5 weight percent ethanol.

A stock solution containing 6.7 mg/mL of a 34 base oligonucleotide was prepared in 10 mmolar histidine, 0.9% NaCl, pH 6.5 aqueous solution.

Aliquots of the lipid stock solution, diluted as needed with ethanol, were hydrated with aliquots of the aqueous oligonucleotide solution, diluted as needed with the aqueous phase, were taken to prepare suspensions of liposomes at the compositions indicated in Fig. 3A.

Each liposome suspension was characterized by measuring the mean particle diameter using QELS just after hydration. The results are shown in Table 3.

**Table 3**

| **Sample** | **Ethanol post hydration (wt%)** | **Total Lipid post hydration (wt%)** | **Oligo post hydration (wt%)** | **Mean diameter 90 degree scatter** | **Mean diameter 30 degree scatter** |
|---|---|---|---|---|---|
| 1 | 36.9 | 2.69 | 0.33 | 814 | 596 |
| 2 | 33.0 | 2.69 | 0.33 | 182.7 | 233 |
| 3 | 29.0 | 2.69 | 0.33 | 114 | 140.3 |
| 4 | 25.1 | 2.69 | 0.33 | 82.1 | 93.9 |
| 5 | 21.2 | 2.69 | 0.33 | 92.6 | 149 |
| 6 | 17.2 | 2.69 | 0.33 | 115.7 | 295 |
| 7 | 13.2 | 2.69 | 0.33 | 209.7 | 311.3 |
| 8 | 11.3 | 2.69 | 0.33 | 274.7 | 423.0 |
| 9 | 9.3 | 2.69 | 0.33 | 334.7 | 524.7 |

Fig. 3B is a plot showing the mean particle diameter in nm as a function of both weight percent lipid and weight percent ethanol.

### Example 4

599.6 mg of partially hydrogenated soy phosphatidylcholine, 257.2 mg of cholesterol, and 214.2 mg of mPEG-DSPE were mixed with 1 mL of ethanol and heated until dissolved. The composition of this lipid stock solution was 57.6 weight percent total lipid in ethanol.

An aqueous phase for hydration was prepared and contained 10 mmolar histidine, 0.9% NaCl, pH 6.5 aqueous solution.

Aliquots of the lipid stock solution, diluted as needed with ethanol, were hydrated with aliquots of the aqueous solution, to prepare suspensions of liposomes at the compositions indicated in Fig. 4A. Each of the compositions were prepared in triplicate.

Each liposome suspension was characterized by measuring the mean particle diameter using QELS just after hydration. The results are shown in Table 4.

**Table 4**

| **Sample** | **Weight Percent EtOH** | **Weight Percent Total lipid** | **Mean particle diameter (nm)** | **Standard deviation** |
|---|---|---|---|---|
| 1 | 7.0 | 0.94 | 413 | 91 |
| 2 | 18.8 | 0.94 | 161 | 21 |
| 3 | 38.5 | 0.94 | 537 | 227 |
| 4 | 4.9 | 3.12 | 1084 | 135 |
| 5 | 16.8 | 3.12 | 569 | 86 |
| 6 | 36.5 | 3.12 | 705 | 192 |
| 7 | 3.5 | 4.69 | 1435 | 76 |
| 8 | 15.3 | 4.69 | 737 | 146 |
| 9 | 35.0 | 4.69 | 735 | 108 |

Fig. 4B is a plot showing the mean particle diameter in nm as a function of both weight percent lipid and weight percent ethanol.

### Example 5

Liposomes were prepared as described in Example 5 except the mPEG-DSPE was omitted for the liposome lipid mixture. Thus, 629.8 mg of partially hydrogenated soy phosphatidylcholine and 272 mg of cholesterol were mixed with 1 mL of ethanol and heated until dissolved. The composition of this lipid stock solution was 53.3 weight percent total lipid in ethanol.

Aliquots of the lipid stock solution, diluted as needed with ethanol, were hydrated with aliquots of the aqueous solution (10 mmolar histidine, 0.9% NaCl, pH 6.5), to prepare suspensions of liposomes at the compositions indicated in Fig. 5A. Each of the compositions were prepared in triplicate.

Each liposome suspension was characterized by measuring the mean particle diameter using QELS just after hydration. The results are shown in Table 5.

**Table 5**

| **Sample** | **Weight percent EtOH** | **Weight percent Total lipid** | **Mean particle diameter (nm)** | **Standard deviation** |
|---|---|---|---|---|
| 1 | 7.1 | 0.87 | 365 | 150 |
| 2 | 18.9 | 0.87 | 495 | 504 |
| 3 | 38.7 | 0.87 | 1596 | 128 |
| 4 | 5.2 | 2.88 | 2891 | 986 |
| 5 | 17.0 | 2.88 | 305 | 68 |
| 6 | 36.7 | 2.88 | 2116 | 407 |
| 7 | 3.8 | 4.33 | 5710 | 516 |
| 8 | 15.6 | 4.33 | 719 | 244 |
| 9 | 35.4 | 4.33 | 2433 | 867 |

Fig. 5B is a plot showing the mean particle diameter in nm as a function of both weight percent lipid and weight percent ethanol.

### Example 6

668.3 mg of partially hydrogenated soy phosphatidylcholine, 222.3 mg of cholesterol, and 222.6 mg of mPEG-DSPE were mixed with 1 mL of ethanol and heated until dissolved. The composition of this lipid stock solution was 55.5 weight percent total lipid in ethanol.

An aqueous phase for hydration was prepared and contained 10 mmolar histidine, 0.9% NaCl, pH 6.5 aqueous solution.

Aliquots of the lipid stock solution, diluted as needed with ethanol, were hydrated with aliquots of the aqueous solution, to prepare suspensions of liposomes at the compositions indicated in Fig. 6A. Each of the compositions were prepared in triplicate.

Each liposome suspension was characterized by measuring the mean particle diameter using QELS just after hydration. The results are shown in Table 6.

**Table 6**

| **Sample** | **Weight percent EtOH** | **Weight percent total lipid** | **Mean particle diameter (nm)** | **Standard deviation** |
|---|---|---|---|---|
| 1 | 7.0 | 0.95 | 411 | 57 |
| 2 | 18.8 | 0.95 | 127 | 9 |
| 3 | 38.6 | 0.95 | 1423 | 135 |
| 4 | 4.9 | 3.2 | 961 | 97 |
| 5 | 16.7 | 3.2 | 418 | 63 |
| 6 | 36.4 | 3.2 | 634 | 41 |
| 7 | 3.4 | 4.8 | 1725 | 127 |
| 8 | 15.2 | 4.8 | 634 | 35 |
| 9 | 35.0 | 4.8 | 837 | 74 |

Fig. 6B is a plot showing the mean particle diameter in nm as a function of both weight percent lipid and weight percent ethanol.

### Example 7

Liposomes were prepared as described in Example 6 except the mPEG-DSPE was omitted for the liposome lipid mixture. Thus, 629.8 mg of partially hydrogenated soy phosphatidylcholine and 272 mg of cholesterol were mixed with 1 mL of ethanol and heated until dissolved. The composition of this lipid stock solution was 54.3 weight percent total lipid in ethanol.

Aliquots of the lipid stock solution, diluted as needed with ethanol, were hydrated with aliquots of the aqueous solution (10 mmolar histidine, 0.9% NaCl, pH 6.5) to prepare suspensions of liposomes at the compositions indicated in Fig. 7A. Each of the compositions were prepared in triplicate.

Each liposome suspension was characterized by measuring the mean particle diameter using QELS just after hydration. The results are shown in Table 7.

**Table 7**

| **Sample** | **Weight percent EtOH** | **Weight percent total lipid** | **Mean particle diameter (nm)** | **Standard deviation** |
|---|---|---|---|---|
| 1 | 7.1 | 0.88 | 222 | 22 |
| 2 | 18.9 | 0.88 | 219 | 24 |
| 3 | 38.6 | 0.88 | 3180 | 331 |
| 4 | 5.1 | 2.9 | 3993 | 1625 |
| 5 | 17.0 | 2.9 | 516 | 299 |
| 6 | 36.7 | 2.9 | 2619 | 1154 |
| 7 | 3.7 | 4.4 | 7014 | 27 |
| 8 | 15.5 | 4.4 | 604 | 106 |
| 9 | 35.3 | 4.4 | 1879 | 322 |

Fig. 7B is a plot showing the mean particle diameter in nm as a function of both weight percent lipid and weight percent ethanol.

### Example 8

### Liposomes Containing dpIUdR

### A. Synthesis of 3',5'-dipalmitoyl-5-iodo-2'-deoxyuridine (dpIUdR)

Palmitoyl chloride was added slowly to a solution of deoxyuridine in pyridine or in pyridine/chloroform and 4-dimethylpyridine as a catalyst. The solution was mixed until a yellow in color and then left to stand overnight for precipitation. The mixture was dissolved in chloforma and washed with 10% aqueous citric acid solution and saturated sodium bicarbonate solution. The chloroform was removed and methanol was added to yield a white precipitate, identified as 3',5'-dipalmitoyl-5-iodo-2'-deoxyuridine (66% yield). The synthetic reaction scheme is shown in Fig. 8.

### B. Liposome Preparation Varying Ethanol Content

The lipids hydrogenated soy phosphatidyl choline (HSPC) and distearoylphosphatidylcholine derivatized with methoxy-polyethyleneglycol (DSPE-mPEG) and dpIUdR in molar ratio of 89/5/6 were dissolved in ethanol at 70°C until complete dissolution was achieved (about 1 hour). This lipid stock solution, after diluting as necessary to maintain a fixed lipid concentration, was hydrated with a 10% sucrose solution at 70°C to vary the ethanol concentration from 8-44 weight percent. Each of the liposome mixtures were stirred for one hour to form liposome suspensions. The liposome particle size of each mixture was determined using quasi-elastic light scattering and the results are shown in Table 8.

### C. Liposome Preparation at 16.5 weight percent Ethanol

The lipids hydrogenated soy phosphatidyl choline (HSPC) and distearoylphosphatidylcholine derivatized with methoxy-polyethyleneglycol (DSPE-mPEG) and dpIUdR in a molar ratios of 89/5/6, 85/5/10, 80/5/15, 70/5/25 and 55/5/40 were dissolved in ethanol at 70°C until complete dissolution was achieved (about 1 hour). The lipid mixtures were hydrated with sufficient 10% sucrose solution at 70°C to achieve a 16.5 weight percentage ethanol content in the hydration mixture (20% (v/v) ethanol). The mixtures were stirred for one hour prior to extruding the mixtures.

The liposome suspensions were extruded through polycarbonate membranes to achieve a uniform size of 100 nm. The suspensions were then diafiltered against 10% sucrose solution to reduce the ethanol concentration to below 400 ppm. The suspensions were then ultrafiltered to above the target drug concentration, assayed for drug concentration and diluted to target by adding 10 mM histidine buffer and adjusting the pH to 6.5.

The liposome particle sizes were measured by quasi-elastic light scattering and the results are shown in Table 9.

## Claims

1. A method for preparing a liposome composition comprising liposomes having a desired size, the method comprising (i) determining an amount of lipid solvent required to achieve the desired liposome size from a profile of liposome size as a function of amount of lipid solvent in a hydration mixture of the lipid solvent and a second solvent with which the lipid solvent is miscible, and (ii) hydrating a vesicle-forming lipid dissolved in said determined amount of lipid solvent with the second solvent to form the hydration mixture, said determined amount of lipid solvent being between 10-50 weight percent.

2. A method according to claim 1 wherein the profile of liposome size as a function of lipid solvent content in the hydration mixture is obtained by measuring the diameter of liposomes in the hydration mixture, and repeating the hydrating and measuring steps at different amounts of lipid solvent to obtain the profile.

3. A method according to claim 1 or claim 2, wherein the lipid solvent is an alcohol.

4. A method according to claim 3, wherein the lipid solvent is methanol, ethanol or butanol.

5. A method according to any preceding claim, wherein the vesicle-forming lipid is a charged vesicle-forming lipid, or a neutral vesicle-forming lipid, and/or is derivatized with a hydrophilic polymer chain.

6. A method according to claim 5, wherein the vesicle-forming lipid is derivatized with a hydrophilic polymer chain, wherein the hydrophilic polymer chain is polyethyleneglycol.

7. A method according to any preceding claim, wherein the second solvent is water.

8. A method according to any preceding claim, wherein a therapeutic agent is hydrated with the lipid solvent or the second solvent.

9. A method according to claim 8, wherein the therapeutic agent is a nucleic acid or a radiosensitizer derivatized with one or more acyl chains.

10. A method according to claim 9, wherein the therapeutic agent is dipalmitoyl 5-iodo-2-dexoyuridine.

## Patentansprüche

1. Verfahren zur Herstellung einer Liposomzusammensetzung, die Liposome mit einer gewünschten Größe umfasst, wobei das Verfahren umfasst: (i) Bestimmung der Menge an Lipidlösungsmittel, die benötigt wird, um die gewünschte Liposomengröße aus einem Profil der Liposomengröße als Funktion der Menge des Lipidtösungsmittels in einem Hydratisierungsgemisch aus dem Lipidlösungsmittel und einem zweiten Lösungsmittel, mit dem das Lipidlösungsmittel mischbar ist, zu erhalten, und (ii) Hydratisieren eines vesikelbildenden Lipids, das in der bestimmten Menge an Lipidlösungsmittel mit dem zweiten Lösungsmittel aufgelöst ist, um das Hydratisierungsgemisch zu bilden, wobei die bestimmte Menge an Lipidlösungsmittel 10-50 Gewichtsprozent beträgt.

2. Verfahren nach Anspruch 1, worin das Profil der Liposomengröße als Funktion des Gehalts an Lipidlösungsmittel im Hydratisierungsgemisch erhalten wird durch Messung des Durchmessers der Liposome im Hydratisierungsgemisch und Wiederholung der Hydratisierungsund Messschritte mit unterschiedlichen Mengen an Lipidlösungsmittel, um das Profil zu erhalten.

3. Verfahren nach Anspruch 1 oder Anspruch 2, worin das Lipidlösungsmittel ein Alkohol ist

4. Verfahren nach Anspruch 3, worin das Lipidlösungsmittel Methanol, Ethanol oder Butanol ist.

5. Verfahren nach einem der vorhergehenden Ansprüche, worin das vesikelbildende Lipid ein geladenes vesikelbildendes Lipid oder ein neutrales vesikelbildendes Lipid ist und/oder mit einer hydrophilen Polymerkette derivatisiert ist.

6. Verfahren nach Anspruch 5, worin das vesikelbildende Lipid mit einer hydrophilen Polymerkette derivatisiert ist und das hydrophile Polymer Polyethylenglycol ist.

7. Verfahren nach einem der vorhergehenden Ansprüche, worin das zweite Lösungsmittel Wasser ist.

8. Verfahren nach einem der vorhergehenden Ansprüche, worin ein therapeutisches Mittel mit dem Lipidlösungsmittel oder dem zweiten Lösungsmittel hydratisiert wird.

9. Verfahren nach Anspruch 8, worin das therapeutische Mittel eine Nudeinsäure oder ein Radioantikörper ist, welche mit einer oder mehreren Acylketten derivatisiert sind.

10. Verfahren nach Anspruch 9, worin das therapeutische Mittel Dipalmitoyl-5-iod-2-desoxyuridin ist.

## Revendications

1. Procédé de préparation d'une composition de liposomes comprenant des liposomes ayant une dimension désirée, le procédé comprenant les opérations consistant à :
(i) déterminer une quantité de solvant de lipide requise pour parvenir à la dimension de liposome désirée à partir d'un profil de dimension de liposome en fonction de la quantité de solvant de lipide dans un mélange d'hydratation du solvant de lipide et d'un second solvant avec lequel le solvant de lipide est miscible ; et
(ii) hydrater un lipide de formation de vésicules, dissous dans ladite quantité déterminée de solvant de lipide avec le second solvant pour former le mélange d'hydratation, ladite quantité déterminée de solvant de lipide étant entre 10-50 pour cent en poids.

2. Procédé selon la revendication 1, dans lequel on obtient le profil de dimension de liposome en fonction de la teneur en solvant de lipide dans le mélange d'hydratation en mesurant le diamètre de liposomes dans le mélange d'hydratation, et en répétant les étapes d'hydratation et de mesure à différentes quantités de solvant de lipide afin d'obtenir le profil.

3. Procédé selon la revendication 1 ou la revendication 2, dans lequel le solvant de lipide est un alcool.

4. Procédé selon la revendication 3, dans lequel le solvant de lipide est le méthanol, l'éthanol ou le butanol.

5. Procédé selon l'une quelconque des revendications précédentes, dans lequel le lipide de formation de vésicules est un lipide de formation de vésicules chargé, ou un lipide de formation des vésicules neutre, et/ou est transformé en dérivé par une chaîne de polymère hydrophile.

6. Procédé selon la revendication 5, dans lequel le lipide de formation de vésicules est transformé en dérivé par une chaîne de polymère hydrophile, dans lequel la chaîne de polymère hydrophile est le polyéthylèneglycol.

7. Procédé selon l'une quelconque des revendications précédentes, dans lequel le second solvant est l'eau.

8. Procédé selon l'une quelconque des revendications précédentes, dans lequel un agent thérapeutique est hydraté par le solvant de lipide ou le second solvant.

9. Procédé selon la revendication 8, dans lequel l'agent thérapeutique est un acide nucléique ou un radiosensibilisateur transformé en dérivé par une ou plusieurs chaînes acyle.

10. Procédé selon la revendication 9, dans lequel l'agent thérapeutique est la dipalmitoyl 5-iodo-2-désoxyuridine.
